# EUROPÄISCHE PATENTANMELDUNG

(11) **EP 0 930 331 A2**
(43) Veröffentlichungstag der Anmeldung: **21.07.1999**
(21) Anmeldenummer: 98123625.0
(22) Anmeldetag: 10.12.1998
(51) Int. Cl.: C08J 7/04, C09D 5/00, C09D 201/02, A61L 15/22, A61L 29/00

(54) **Bioaktive und hydrophile Beschichtung von polymeren Substraten**

(30) Priorität: 14.01.1998 DE 19801038; 23.06.1998 DE 19827871
(71) Anmelder: HÜLS AKTIENGESELLSCHAFT, 45764 Marl (DE)
(72) Erfinder: Anders, Christine Dr., 45721 Haltern (DE); Spannenkrebs, Dorothea, 45768 Marl (DE)
(74) Vertreter: Olbricht, Gerhard Dr.

(57) **Zusammenfassung**

Die Erfindung betrifft ein Verfahren zur hydrophilen und bioaktiven Beschichtung von polymeren Substraten, bei dem man ein hydrophiles oder hydrophiliertes polymeres Substrat mit einem Polyalkylenimin als Haftvermittler oder mit einem Ammoniak-plasma behandelt und auf das behandelte Substrat ein (a) Carboxyl- und/oder Carboxylatgruppen und/oder (b) Sulfonsäure und/oder Sulfonatgruppen und/oder (c) saure Schwefelsäureester- und/oder -sulfatgruppen enthaltendes hydrophiles bioaktives Beschichtungspolymer aufbringt. Die beschichteten Substrate eignen sich zur Verwendung für technische, medizintechnische, hygienische oder biotechnische Zwecke.

## Beschreibung

Die Erfindung betrifft ein Verfahren zur hydrophilen und bioaktiven Beschichtung von polymeren Substraten, bei dem die Beschichtung über einen Haftvermittler (Primer) fest an das Substrat gebunden ist. Die Erfindung betrifft weiterhin Erzeugnisse mit derart beschichteten Substraten sowie deren Verwendung für technische. medizinische oder biotechnische Zwecke. Die erfindungsgemäßen bioaktiven Beschichtungen sind hydrophil und insbesondere bakterienabweisend.

### 1. Stand der Technik

Die Ansiedelung und Vermehrung von Bakterien auf Oberflächen ist eine in der Regel unerwünschte Erscheinung, die häufig mit nachteiligen Folgen verbunden ist. So können in der Trinkwasser- und Getränketechnik Bakterienpopulationen zu einer gesundheitsgefährdenden Qualitätsminderung führen. Bakterien auf oder in Verpackungen bewirken häufig den Verderb von Lebensmitteln oder verursachen sogar Infektionen bei dem Verbraucher. In steril zu betreibenden biotechnischen Anlagen stellen systemfremde Bakterien ein erhebliches prozeßtechnisches Risiko dar, Solche Bakterien können mit Rohstoffen eingetragen werden oder bei mangelhafter Sterilisation in allen Anlageteilen zurückbleiben Teile der Bakterienpopulation können sich durch Adhäsion dem normalen Flüssigkeitsaustausch beim Spülen und Reinigen entziehen und sich im System vermehren.

Weiterhin sind Bakterienansiedelungen in Wasseraufbereitungsanlagen (z.B. zur Entsalzung durch Membranen) oder auch in Behältern bekannt, die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen.

Besondere Bedeutung kommt dem Schutz vor Bakterienanhaftung und -ausbreitung in der Ernährung, der Pflege, hier insbesondere in der Altenpflege, und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung, z.B. in Bädern. Solche Einrichtungen sind bevorzugte Lebensräume für Bakterien, ebenso wie bestimmte Oberflächen in Bereichen mit hohem Publikumsverkehr, so z.B. in öffentlichen Verkehrsmitteln. Krankenhäusern, Telefonzellen, Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und bei häuslicher Pflege.

Besondere Sorgfalt bedarf die Verwendung medizinischer Gegenstände und Geräte bei medizinischen Untersuchungen. Behandlungen und Eingriffen, vor allem dann, wenn derartige Geräte oder Gegenstände mit lebendem Gewebe oder mit Körperflüssigkeiten in Kontakt kommen. Im Falle von Langzeit- oder Dauerkontakten, beispielsweise bei Implantaten, Kathetern, Stents, Herzklappen und Herzschrittmachern, können Bakterienkontaminationen zu einem lebensbedrohenden Risiko für den Patienten werden.

Es wurde bereits auf vielfältige Weise versucht, die Ansiedelung und Ausbreitung von Bakterien auf Oberflächen zu unterbinden. In J. Microbiol. Chemoth. **31** (1993), 261-271 beschreiben S.E.Tebbs und T.S.J.Elliott lackartige Beschichtungen mit quaternären Ammoniumsalzen als antimikrobiell wirkenden Komponenten. Es ist bekannt, daß diese Salze von Wasser, wäßrigen oder anderen polaren Medien sowie von Körperflüssigkeiten aus dem Beschichtungsmaterial herausgelöst werden und ihre Wirkung somit nur von kurzer Dauer ist. Dies gilt gleichermaßen für die Einarbeitung von Silbersalzen in Beschichtungen, so beschrieben in WO 92/18098.

T. Ouchi und Y. Ohya beschreiben in Progr.Polym.Sci. **20** (1995), 211 ff., die Immobilisierung von bakteriziden Wirkstoffen auf Polymeroberflächen durch kovalente Bindung oder ionische Wechselwirkungen. Häufig sind in solchen Fällen die keimtötenden Wirkungen gegenüber dem reinen Wirkstoff deutlich reduziert. Heteropolare Bindungen erweisen sich oft als nicht hinreichend stabil. Darüber hinaus führt die Keimabtötung in der Regel zu unerwünschten Ablagerungen auf den Oberflächen, die die weitere bakterizide Wirkung maskieren und die Grundlage für eine nachfolgende Bakterienbesiedelung bilden.

W. Kohnen et al. berichten in ZBl. Bakt. Suppl. 26, Gustav Fischer Verlag, Stuttgart-Jena-New York, 1994, Seiten 408 bis 410, daß die Adhäsion von Staphylococcus epidermidis auf einem Polyurethanfilm vermindert wird, wenn der Film durch eine Glimmentladung in Gegenwart von Sauerstoff vorbehandelt und mit Acrylsäure gepfropft wird.

Wie erwähnt, ist es wichtig, daß bei Verwendung medizinischer Gegenstände und Geräte bei medizinischen Untersuchungen, Behandlungen und Eingriffen eine Bakterienkontamination dieser Gegenstände und Geräte verhindert wird. Bei manchen dieser Gegenstände und Geräte, die mittel- oder langfristig mit lebendem Gewebe oder Körperflüssigkeiten in Kontakt kommen, ist zudem eine Haftung und Ausbreitung von körpereigenen Zellen ausgesprochen unerwünscht. So sind Zellbesiedelungen bei mittelfristig intrakorporal applizierten Kathetern ebenso schädlich wie bei langfristig implantierten Stents oder Herzklappen.

Weiterhin kann die Transparenz von Intraokularlinsen infolge von Zellbesiedelung nach der Implantation kontinuierlich abnehmen. Eine Reihe von Verfahren zielt darauf ab, eine Zellbesiedelung, beispielsweise durch die Einarbeitung bestimmter Metalle oder Metallsalze in die Halterung der Intraokularlinse zu vermeiden, wobei die Wirkung jedoch meist unvollständig und nicht nachhaltig ist. Auch in WO 94/16648 wird ein Verfahren beschrieben, durch das die Proliferation von Zellen auf der Oberfläche von implantierten Okularlinsen aus Polymermaterial verhindert werden soll.

Gemäß EP 0 431 213 sollen Polymere mit zellabweisenden Eigenschaften ausgestattet werden, indem ihre Oberfläche mit starken Mineralsäuren hydrophiliert wird. Die nachträgliche chemische Modifikation von Polymeroberflächen ist jedoch meist nicht gleichmäßig. Es bleiben in der Regel nicht oder nicht ausreichend behandelte Stellen zurück, die Ausgangspunkte für eine Zellbesiedelung bilden. Weiterhin sind die zellabweisenden Eigenschaften der so behandelten Oberflächen häufig nicht dauerhaft.

Andererseits sind für bestimmte Verwendungen Gegenstände mit Oberflächen erwünscht, die bakterienabweisend sind, aber die Besiedelung mit Zellen fördern. Das gilt z.B. für eine Reihe von Geräten für medizinische Untersuchungen, Behandlungen und Eingriffe und ebenso für manche Prothesen, die in das Gewebe einwachsen sollen, in das sie implantiert wurden. Solche Geräte und Prothesen, z.B. künstliche Hüftgelenke oder Zähne, bestehen häufig aus polymerummantelten Materialien, wie Titan.

Schließlich müssen Materialien für Geräte und Vorrichtungen, die mit Körperflüssigkeiten, wie Blut oder Lymphe, oder mit Gewebe in Kontakt kommen, verträglich für ihre fremde Umgebung sein. Insbesondere ist Blutverträglichkeit eine wichtige erwünschte Eigenschaft. Die Materialien müssen also möglichst ausgeprägte antithrombische Eigenschaften haben. Solche Materialien werden z.B. in den deutschen Patentanmeldungen 192 20 369.8 (O.Z. 5195) und 198 01 040.0 (O.Z. 5281) beschrieben.

Es gibt also verschiedene, einander teilweise ausschließende Anforderungen an die bioaktiven Eigenschaften der Oberfläche von Polymeren, die für medizinische Verwendungen bestimmt sind. Sie sollen stets bakterienabweisend und verträglich mit Körperflüssigkeiten und Gewebe sein, aber wahlweise zellproliferationshemmend oder -fordernd wirken.

Der vorliegenden Erfindung lag die Aufgabe zugrunde, ein verbessertes Verfahren zur Beschichtung von Oberflächen zu entwickeln, mit dem die Oberflächen nachhaltig hydrophil und bioaktiv, nämlich bakterienabweisend und hämokompatibel (antithrombisch) sowie verträglich für Körperflüssigkeiten und Gewebe und wahlweise zellproliferationshemmend oder -fördernd beschichtet werden können, ohne daß die mechanischen Eigenschaften der behandelten Materialien dadurch verändert werden oder sonstige erhebliche Nachteile eintreten.

### 2. Kurzbeschreibung der Erfindung

Ein Gegenstand der Erfindung ist dementsprechend ein Verfahren zur hydrophilen und bioaktiven Beschichtung von polymeren Substraten, bei dem man ein hydrophiles oder hydrophiliertes polymeres Substrat mit einem Polylkylenimin als Haftvermittler (Primer) behandelt und auf das behandelte Substrat ein (a) Carboxyl- und/oder Carboxylatgruppen und/oder (b) Sulfonsäure- und/oder Sulfonatgruppen und/oder (c) saure Schwefelsäureester und/oder -sulfatgruppen enthaltendes hydrophiles bioaktives Beschichtungspolymer aufbringt.

Bei einer alternativen Variante des Verfahrens wird das polymere Substrat statt mit einem Polyalkylenimin mit einem Ammoniak-Plasma behandelt, wobei das polymere Substrat nicht hydrophil oder hydrophiliert sein muß.

Ein gemeinsames Merkmal beider Verfahrensvarianten besteht darin, daß durch die Behandlung des Substrats basische Gruppen geschaffen werden, die die Haftung des hydrophilen bioaktiven Beschichtungspolymers verbessern.

Ein anderer Gegenstand der Erfindung sind Erzeugnisse, die aus einem nach den erfindungsgemäßen Verfahren hydrophil und bioaktiv beschichteten polymeren Substrat bestehen oder ein solches Substrat enthalten.

Ein weiterer Gegenstand der Erfindung ist die Verwendung der erfindungsgemäßen Erzeugnisse für technische, medizintechnische, hygienische oder biotechnische Zwecke.

Das Beschichtungspolymer muß mindestens eine der genannten Gruppen aufweisen. Die sauren Gruppen können durch Behandlung mit einer Base, wie Natronlauge, ohne weiteres ganz oder teilweise in die Salzform überführt werden. So können z.B. Carboxyl- und Carboxylatgruppen nebeneinander vorliegen. Neben diesen Gruppen oder an deren Stelle können aber auch Schwefel enthaltende Gruppen vorhanden sein. Als Beispiele seien Beschichtungspolymere genannt, die nur Schwefelsäureestersulfatgruppen (d.h. neutralisierte Schwefelsäureester-Gruppen) aufweisen, oder solche, die Carboxyl- und/oder Carboxylatgruppen sowie Sulfonatgruppen enthalten.

Wenn das hydrophile oder hydrophilierte polymere Substrat mit einem Polyalkylenimin vorbehandelt wird, ist dessen Haftung auf dem Substrat besonders fest, wenn das Substrat saure Gruppen, beispielsweise Carboxyl-, Sulfonsäure- oder saure Schwefelsäureester-Gruppen, aufweist. Möglicherweise geht diese besonders feste Haftung auf ionische Bindungen zurück, die sich zwischen Polyalkylenimin und Substrat ausbilden. Die sauren Gruppen können als Bestandteile von Monomeren einpolymerisiert worden sein oder durch eine Oberflächenbehandlung von Standardpolymeren ohne einpolymerisierte sauren Gruppen geschaffen werden. Solche Oberflächenbehandlungen, die zugleich hydrophilierend wirken, sind in der Folge beschrieben. Die Beschichtung von hydrophilen oder hydrophilierten, saure Gruppen enthaltenden polymeren Substraten mit dem Polyalkylenimin als Haftvermittler für die bioaktive Schicht ist eine bevorzugte Ausführungsform des Verfahrens nach der Erfindung. Das Polyalkylenimin haftet aber selbst dann überraschend fest auf dem hydrophilen oder hydrophilierten polymeren Substrat, wenn dieses keine sauren Gruppen aufweist.

Wenn das hydrophile, bioaktive Beschichtungspolymer saure Gruppen, enthält, wird diese Schicht ihrerseits ionisch und damit besonders fest an das mit einem Polyalkylenimin bzw. mit Ammoniak-Plasma behandelte polymere Substrat gebunden. Aber auch lediglich Carboxylat-, Sulfonat- oder saure Schwefelsäureestersulfat-Gruppen enthaltende hydrophile, bioaktive Beschichtungspolymere haften bemerkenswert fest auf dem mit Polyalkylenimin bzw. mit Ammoniak-Plasma vorbehandelten polymeren Substrat.

In EP 0 603 987 A1 werden eine hydrophil-anionische oder hydrophilkationische Oberflächenschicht auf einem hydrophoben Polymer, wie Polysulfon, Polyamid oder Polyester, sowie ein Verfahren zu ihrer Herstellung beschrieben. Die Schicht besteht aus einem Komplex, der aus einem oder mehreren wasserlöslichen kationischen Polymeren und bzw. oder einem oder mehreren waserlöslichen kationenaktiven Tensiden und einem oder mehreren anionischen Polymeren und/oder einem oder mehreren anionenaktiven Tensiden entsteht, wobei das Verhältnis der kationischen zu den anionischen Gruppen im Komplex ungleich 1 ist. Die aufgebrachte Schicht wird als adsorptiv haftend beschrieben. Zu den aufgeführten kationischen Polymeren zählt auch Polyethylenimin. Weiterhin sollen fakultativ auch ionische Wirkstoffe gebunden werden können. Im Gegensatz zu der Lehre der EP 0 603 987 A1 geht jedoch das Verfahren nach der Erfindung von hydrophilen oder hydrophilierten Polymeren aus. Dadurch wird eine Beschichtung erzeugt, die erheblich fester haftet als eine Beschichtung auf einem hydrophoben polymeren Substrat unter sonst vergleichbaren Bedingungen. Das aufgebrachte Polyalkylenimin haftet vorzugsweise ionisch statt adsorptiv und damit besonders fest, und auf das Polyalkylenimin wird ein mindestens eine der genannten Gruppen enthaltendes Beschichtungspolymer aufgebracht, das wiederum ionisch und damit besonders fest haftet, sofern es saure Gruppen enthält.

### 3. Vorteile der Erfindung

Die erfindungsgemäß beschichteten Substrate zeigen eine bemerkenswerte Kombination vorteilhafter Eigenschaften und eine hervorragende physiologische Verträglichkeit. Sie sind insbesondere gut blutverträglich und vermindern die Adhäsion und Vermehrung von Bakterien in einem hohen Maße auch über längere Zeit. Von dieser Wirkung betroffene Bakterien sind u.a. Staphylococcus aureus, Staphylococcus epidermidis, Streptococcus pyogenes, Klebsiella pneumoniae, Pseudomonas aeroginosa und Escherichia coli. Gleichzeitig wird meistens auch die Proliferation von Zellen inhibiert, beispielsweise von Fibroblasten und Endothelzellen, wie menschlichen Nabelschnurzellen. Die besonderen Bedingungen, unter denen eine Beschichtung bakterienabweisend, aber zellproliferations fördernd sind, werden später erläutert. Polyalkylenimine sind zumindest in den angewandten Konzentrationen nicht toxisch, so daß die beschichteten Substrate auch für medizintechnische Anwendungen geeignet sind. Die Behandlung der polymeren Substrate mit Ammoniak-Plasma begründet zumindest dann keine toxikologischen Risiken, wenn nach der Beschichtung etwa gebildete toxische Monomere oder ablösbare Fragmentmoleküle durch Extraktion entfernt werden.

### 4. Beschreibung der Erfindung

### 4.1 Die polymeren Substrate

Bei der Variante des erfindungsgemäßen Verfahren mit einem Polyalkylenimin als Primer geht man von Polymeren aus, die hydrophil sind, d.h. von ihrer Herstellung her hydrophile Gruppen im Bulk enthalten, oder oberflächig hydrophiliert wurden.

Bei der alternativen Variante mit Vorbehandlung mittels Ammoniak-Plasma müssen dagegen die polymeren Substrate nicht hydrophil oder hydrophiliert, können also hydrophob sein. Nicht hydrophile polymere Substrate werden jedoch durch die Vorbehandlung hydrophiliert.

Die polymeren Substrate können mannigfache geometrische Formen aufweisen, beispielsweise Platten, Folien, Rohre oder Schläuche sein, je nach der beabsichtigten Verwendung des betreffenden Erzeugnisses.

### 4.1.1 Hydrophile polymere Substrate

Bei den hydrophilen Polymeren handelt es sich, im Gegensatz zu den später diskutierten nachträglich hydrophilierten Standardpolymeren, um Spezialprodukte, die jedoch zum Teil im Handel erhältlich sind. Geeignete hydrophile Polymere sind Homopolymere aus gegebenenfalls zugleich saure Gruppen tragenden hydrophilen Vinylmonomeren oder aber hinreichend hydrophile Copolymere aus gegebenenfalls zugleich saure Gruppen tragenden hydrophilen Vinylmonomeren und hydrophoben Vinylmonomeren. Von den letzteren seien z.B. Vinylchlorid, Ethylen, Propylen, 1-Buten, 1-Octen, Isopren, Styrol, α-Methylstyrol, 2- und 4-Vinyltoluol, 2-Ethylhexylacrylat, Tetrafluorethylen, Methylmethacrylat, Methacrylamid, 1,3-Butadien, Vinylpyridin, Vinylidenchlorid und Vinylacetat genannt.

Geeignete hydrophile Vinylmonomere sind bei 20°C in Wasser zu mindestens 1 Gewichtsprozent, vorteilhaft zu mindestens 10 Gewichtsprozent und insbesondere zu mindestens 40 Gewichtsprozent löslich, jeweils bezogen auf die gesamte Lösung. Von den geeigneten hydrophilen Vinylmonomeren seien beispielsweise genannt: Acrylsäure und deren Derivate, z.B. Acrylamid, N,N,-Dimethylacrylamid, Acrylnitril, Methylacrylat, 2-Hydroxyethylacrylat, 2-Hydroxypropylacrylat, 2-Methoxyethylacrylat; 2-Ethoxyethylacrylat, 4-Hydroxybutylacrylat und 1,4-Butandioldiacrylat, sowie Methacrylsäure und deren entsprechende Derivate; Carbonsäurevinylderivate, wie Vinylacetat, N-Vinylacetamid und N-Vinylpyrrolidon; Vinylsulfonsäuren und deren Alkalisalze, wie Natriumvinylsulfonat; Alkenylarylsulfonsäuren und deren Alkalisalze, wie o- und p-Styrolsulfonsäure und Natriumstyrolsulfonat, Vinylether, wie Vinylmethylether, Vinylethylether, Vinylglycidylether, Diethylenglykoldivinylether und Vinyl-n-butylether; Vinylketone, wie Vinylmethylketon, Vinylethylketon und Vinyl-n-propylketon; Vinylamine, wie N-Vinylpyrrolidin; Polyalkylenverbindungen mit endständigen Allyl-, Vinyl -, Acryl- oder Methacrylgruppen, wie Ethoxytetraethoxyethylacrylat oder -methacrylat, n-Propoxydodecaethylenoxyethylvinylether, Polyethylenglykolmonoacrylate mit Molgewichten von 600 oder 1.200, Poly(ethylen/propylen)glykolmonomethacrylate mit Molgewichten von 400 und 800 sowie Allyloxyoctapropylenoxyethanol; Zuckerderivate, wie vinylsubstituierte Arabinose oder acryloylierte Hydroxypropylzellulose; und funktionalisierte Polyalkylenglykole, wie Triethylenglykoldiacrylat oder Tetraethylenglykoldiallylether.

Für die Herstellung von hydrophilen Copolymeren aus hydrophilen und hydrophoben Comonomeren sollten die hydrophilen Vinylmonomeren zumindest in einer solchen Menge eingesetzt werden, daß der Kontaktwinkel, gemessen bei 25°C nach der Methode von R.J.Good et al., die später erläutert werden wird, <40°, vorteilhaft <30° beträgt. Ein solches Copolymer wird als hydrophil im Sinne der Erfindung angesehen. Homo- oder Copolymere, die ausschließlich aus hydrophilen Vinylmonomeren aufgebaut sind, genügen dieser Bedingung ohnehin. Der Anteil der sauren Gruppen sollte gegebenenfalls so hoch sein, daß der kationische Primer wirksam ionisch gebunden wird, was schon bei geringen Konzentrationen der Fall ist. Zweckmäßig beträgt der molare Anteil der Vinylmonomeren mit sauren Gruppen im Copolymer mindestens 10 Molprozent.

### 4.1.2 Hydrophilierte polymere Substrate

Eine größere Zahl von Optionen hinsichtlich der mechanischen und sonstigen Eigenschaften des Polymersubstrats ist gegeben, wenn man von einem der in großer Vielzahl verfügbaren hydrophoben Standardpolymeren ausgeht und dieses hydrophiliert. Zu den geeigneten Standardpolymeren zählen Homo- und Copolymere, beispielsweise Polyolefine oder Polydiene, wie Polyethylen, Polypropylen, Polyisobutylen, Polybutadien, Polyisopren, natürliche Kautschuke und Polyethylenco-propylen; halogenhaltige Polymere, wie Polyvinylchlorid, Polyvinylidenchlorid, Polychloropren, Polytetrafluorethylen und Polyvinylidenfluorid; Polymere und Copolymere aus vinylaromatischen Monomeren, wie Polystyrol, Polyvinyltoluol, Polystyrol-co-vinyltoluol, Polystyrol-co-acrylnitril, Polystyrol-co-butadien-co-acrylnitril, Poly(styrol-coethylen-co-1-buten); Poly(styrol-co-ethylen-co-2-buten). Polykondensate, beispielsweise Polyester, wie Polyethylenterephthalat und Polybutylenterephthalat; Polyamide, wie Polycaprolactam, Polylaurinlactam und das Polykondensat aus Adipinsäure und Hexamethylendiamin; Polyetherblockamide, z.B. aus Laurinlactem und Polyethylenglykol mit durchschnittlich 8, 12 oder 16 Ethylenoxygruppen; weiterhin Polyurethane, Polyether, Polycarbonate, Polysulfone, Polyetherketone, Polyesteramide und -imide, Polyacrylnitril, Polyacrylate und -methacrylate sowie Silicone. Auch Blends aus zwei oder mehr Polymeren oder Copolymeren lassen sich nach dem Verfahren hydrophilieren, ebenso wie Kombinationen aus verschiedenen Kunststoffen, die durch Verkleben, Verschweißen oder Schmelzen miteinender verbunden sind, einschließlich der Übergangsbereiche.

Die Oberflächen der Substrate können nach einer Reihe von Methoden hydrophiliert und in der Mehrzahl der Fälle gleichzeitig mit sauren Gruppen ausgestattet werden. Zweckmäßig werden sie zuvor in bekannter Weise mittels eines Lösungsmittels von anhaftenden Ölen. Fetten oder anderen Verunreinigungen befreit. Die folgenden Hydrophilierungsmethoden seien erwähnt:
(1) Die Hydrophilierung von Standardpolymeren ohne UV-strahlungssensitive Gruppen kann vorteilhaft durch UV-Strahlung, z.B. im Wellenlängenbereich von 100 bis 400 nm, vorzugsweise von 125 bis 310 nm erfolgen. Besonders gute Ergebnisse wurden mit weitgehend monochromatischer, kontinuierlicher Strahlung erzielt, wie sie z.B. von Excimer-UV-Strahlern (Fa. Heraeus, Kleinostheim, BR Deutschland) beispielsweise mit F₂, Xe₂, ArF, XeCl, KrCl und KrF als Lampenmedium, erzeugt wird. Aber auch andere Strahlungsquellen, wie Quecksilberdampflampen mit breitem Strahlungsspektrum und Strahlungsanteilen im sichtbaren Bereich, sind geeignet, sofern sie erhebliche Strahlungsanteile in den genannten Wellenlängenbereichen emittieren. Es hat sich gezeigt, daß die Anwesenheit kleiner Mengen an Sauerstoff vorteilhaft ist. Die bevorzugten Sauerstoffpartialdrücke liegen zwischen 2x10⁻⁵ und 2x10⁻² bar. Man arbeitet beispielsweise in einem Vakuum von 10⁻⁴ bis 10⁻¹ bar oder unter Verwendung eines Inertgases, wie Helium, Stickstoff oder Argon, mit einem Sauerstoffgehalt von 0,02 bis 20 Promille. Die optimale Bestrahlungsdauer hängt von dem polymeren Substrat, der Zusammensetzung des umgebenden Gasmediums, der Wellenlänge der Strahlen sowie der Leistung der Strahlenquelle ab und läßt sich durch orientierende Vorversuche unschwer ermitteln. Im allgemeinen bestrahlt man die Substrate 0,1 Sekunde bis 20 Minuten lang, insbesondere 1 Sekunde bis 10 Minuten. Bei diesen kurzen Bestrahlungszeiten erwärmt sich das polymere Substrat nur in geringem Maße, und es treten selbst bei Strahlen mit Wellenlängen am harten Ende des genannten weiteren Bereichs bei entsprechend kurz bemessenen Bestrahlungszeiten keine unerwünschten Reaktionen auf, die zu Schäden an den exponierten Oberflächen führen könnten.
(2) Die Hydrophilierung kann auch durch ein Hochfrequenz- oder Mikrowellenplasma (Hexagon, Fa. Technics Plasma, 85551 Kirchheim, Deutschland) in Luft, Sauerstoff-, Stickstoff- oder Argonatmosphäre erreicht werden. Die Expositionszeiten betragen im allgemeinen 30 Sekunden bis 30 Minuten, vorzugsweise 2 bis 10 Minuten. Der Energieeintrag liegt bei Laborgeräten zwischen 100 und 500W, vorzugsweise zwischen 200 und 300W.
(3) Weiterhin lassen sich auch Korona-Geräte (Fa. SOFTAL. Hamburg, Deutschland) zur Hydrophilierung verwenden. Die Expositionszeiten betragen in diesem Falle in der Regel 1 Sekunde bis 10 Minuten, vorzugsweise 1 bis 60 Sekunden.
(4) Die Hydrophilierung durch Elektronen- oder gamma-Strahlen (z.B. aus einer Kobalt-60-Quelle) ermöglicht kurze Expositionszeiten, die im allgemeinen 0,1 bis 60 Sekunden betragen.
(5) Beflammungen von Oberflächen führen ebenfalls zu deren Hydrophilierung, Geeignete Geräte, insbesondere solche mit einer Barriere-Flammenfront, lassen sich auf einfache Weise bauen oder beispielsweise beziehen von der Fa. ARCOTEC, 71297 Mönsheim, Deutschland. Sie können mit Kohlenwasserstoffen oder Wasserstoff als Brenngas betrieben werden. In jedem Fall muß eine schädliche Überhitzung des Substrats vermieden werden, was durch innigen Kontakt mit einer gekühlten Metallfläche auf der von der Beflammungsseite abgewandten Substratoberfläche leicht erreicht wird. Die Hydrophilierung durch Beflammung ist dementsprechend auf verhältnismäßig dünne, flächige Substrate beschränkt. Die Expositionszeiten belaufen sich im allgemeinen aus 0.1 Sekunde bis 1 Minute, vorzugsweise 0.5 bis 2 Sekunden, wobei es sich ausnahmslos um nicht leichtende Flammen handelt und die Abstände der Substratoberflächen zur äußeren Flammenfront 0.2 bis 5 cm, vorzugsweise 0.5 bis 2 cm betragen.
(6) Weiterhin lassen sich Substratoberflächen auch durch Behandlung mit starken Säuren oder Basen hydrophilieren. Von den geeigneten starken Säuren seien Schwefelsäure, Salpetersäure und Salzsäure genannt. Man kann z.B. Polyamide 5 Sekunden bis 1 Minute mit konzentrierter Schwefelsäure bei Raumtemperatur behandeln. Als starke Basen eignen sich besonders Alkalimetallhydroxide in Wasser oder einem organischen Lösemittel. So kann man z.B. verdünnte Natronlauge 1 bis 60 Minuten bei 20 bis 80°C auf die Substrate einwirken lassen. Alternativ können z.B. Polyamide aktiviert werden, indem man 2 %iges KOH in Tetrahydrofuran 1 Minute bis 30 Minuten auf die Substratoberfläche einwirken läßt. Nach Hydrophilierung mit einer starken Base weist das polymere Substrat natürlich keine sauren Gruppen auf, es kann jedoch durch Behandlung mit einer Säure sauer eingestellt werden.

In manchen Fällen. z.B. bei hochhydrophoben Polymeren, kann es empfehlenswert sein, die Substratoberfläche durch eine Kombination aus zwei oder mehr der genannten Methoden zu aktivieren. Die bevorzugte Hydrophilierungsmethode ist die mit UV-Strahlung gemäß Ziffer (1). Was auch immer im einzelnen bei den beschriebenen Behandlungen auf der molekularen Ebene geschieht, so ist das Ergebnis doch eindeutig. Die Hydrophilierung läßt sich durch die Veränderung des Kontaktwinkels nachweisen, der bei Bestimmung nach der erwähnten Methode von R.J.Goods et al. bei 25°C <40°, vorteilhaft <30° betragen sollte. Die entstandenen sauren Gruppen sind durch Titration mit Alkalilauge nachweisbar.

### 4.1.3 Nicht hydrophile (oder hydrophobe) polymere Substrate

Solche Substrate zeigen nach der erwähnte Methode von R.J.Good einen Kontaktwinkel von >40°; sie können erfindungsgemäß eingesetzt werden, wenn eine Vorbehandlung mit Ammoniak-Plasma gewählt wird. Zu den geeigneten Polymeren zählen die unter 4.1.2 erwähnten Standardpolymere, die Homo- und Copolymere sein können, nämlich Polyolefine oder Polydiene, halogenhaltige Polymere, Polymere und Copolymere aus vinylaromatischen Monomeren. Wie bereits erläutert, werden die nicht hydrophilen polymeren Substrate durch die Behandlung mit Ammoniak-Plasma hydrophiliert und wird die Haftung des hydrophilen bioaktiven Beschichtungspolymers auf dem behandelten polymeren Substrat verbessert.

### 4.2 Polyalkylenimin als Haftvermittler

Die Polyalkylenimine werden durch Polymerisation der monomeren Alkylenimine (oder Aziridine) hergestellt und enthalten in wechselnden Mengenverhältnissen primäre, sekundäre und tertiäre Aminogruppen sowie geradkettige, verzweigtkettige und vernetzte Anteile. Das bekannteste Polyalkylenimin ist Polyethylenimin, das im Handel z.B. als ca. 50-gewichtsprozentige wässerige Lösung oder als praktisch wasserfreies Produkt erhältlich ist. Ebenfalls für die vorliegende Erfindung geeignet sind Polypropylenimin sowie Poly(co-ethylenimin-co-propylenimin). Die Polyalkylenimine können zahlendurchschnittliche Molekulargewichte bis zu mehreren Millionen aufweisen. Das Molekulargewicht sollte im Interesse einer guten Haftung >10.000 betragen und liegt vorteilhaft zwischen 10.000 und 2.000.000. Das hydrophile oder hydrophilierte Polymersubstrat wird zweckmäßig mit einer 0,5 bis 20-gewichtsprozentigen Lösungen des Polyethylenimins behandelt, vorteilhaft etwa 30 Sekunden bei Raumtemperatur oder mäßig erhöhter Temperatur bis zu etwa 60°C. Das bevorzugte Lösemittel ist Wasser, gegebenenfalls mit untergeordneten Anteilen an einem niederen Alkohol, wie Methanol oder Ethanol. Nach der Behandlung kann man das Polymersubstrat zunächst trocknen oder aber gleich die Carboxylund/oder Carboxylatgruppen sowie gegebenenfalls Sulfonsäure und/- oder Sulfonatgruppen enthaltende hydrophile bioaktive Schicht aufbringen.

### 4.3 Behandlung des polymeren Substrats mit Ammoniak-Plasma

Die Verfahrensvariante mit Ammoniak-Plasma zur Erzeugung der wie eine Primerschicht wirkenden basischen Gruppen hat den Vorteil, daß man keine hydrophilen oder hydrophilierten polymeren Substrate benötigt, sondern von nicht hydrophilen (also hydrophoben) Standardpolymeren ausgehen kann. Man kann allerdings auch die erwähnten hydrophilen oder hydrophilierten Polymere einsetzen und erzielt dann nicht selten eine noch weiter verbesserte Haftung der hydrophilen bioaktiven Beschichtung.

Für die Behandlung mit Ammoniak eignen sich sowohl Hochfrequenzplasma (im Kiloherzbereich) als auch Mikrowellenplasma (im Gigaherzbereich). Man evakuiert die Behandlungskammer und stellt dann einen bestimmten Ammoniakdruck ein, beispielsweise von 10 bis 500 Pa, vorzugsweise von 20 bis 180 Pa. Der Plasmagenerator kann in einem breiten Leistungsspektrum arbeiten. z.B. von einigen hundert Watt, wie 200 Watt, bis zu einigen Kilowatt, z.B. 10 Kilowatt. Die Behandlungsdauer kann ebenfalls innerhalb weiter Grenzen schwanken, z.B. 10 Sekunden bis zu 30 Minuten betragen. Nach dem Ende der Behandlung wird das Ammoniakgas abgepumpt oder mit Luft verdrängt, und die Behandlungskammer wird zweckmäßig mit Luft gespült. Alternativ kann das Ammoniak-Plasma auch in strömendem Ammoniakgas erzeugt werden, indem man laufend Ammoniak zuführt und abzieht und dabei auf einen Druck in den genannten Bereichen einregelt. Eine optimale Kombination von Frequenz, Leistung, Behandlungsdauer und Ammoniakdruck läßt sich für eine gegebenen Beschichtungsaufgabe durch orientierende Versuche unschwer ermitteln

Durch die Plasmabehandlung entstehen auf der Polymeroberfläche reaktive Gruppen, insbesondere Aminogruppen. Bei Anwesenheit von Sauerstoff werden auch sauerstoffhaltige reaktive Gruppen gebildet. möglicherweise Hydroxyl-, Carboxyl-, Carbonyl- und/oder Hydroperoxygruppen. Für die Haftunng der späteren Beschichtung dürften jedoch die Aminogruppen am wichtigsten sein, da Aminogruppen bekanntlich leichter mit Isocyanatgruppen reagieren als die genannten sauerstoffhaltigen reaktiven Gruppen. Die Aminogruppen können bei Stickstoff-freien Substraten mittels ESCA und bei allen Substraten mittels Säuretitration nachgewiesen werden.

### 4.4 Hydrophile bioaktive Schicht

Auf dem mit einem Polyalkylenimin oder Ammoniak-Plasma behandelten polymeren Substrat haftet die hydrophile bioaktive Beschichtung ionisch, nämlich durch Ausbildung von Ammoniumcarboxylat-, Ammoniumsulfonat- oder Ammoniumsulfat-Strukturen, sofern das Beschichtungspolymer Carboxyl-, Sulfonsäure- oder saure Schwefelsäureester-Gruppen enthält. Die Beschichtung kann aufgebracht werden, indem man auf das behandelte polymere Substrat eine Lösung, vorteilhaft eine wäßrige Lösung eines entsprechenden hydrophilen bioaktiven Beschichtungspolymers einwirken läßt, das die genannten Gruppen enthält. Die gegebenenfalls vorhandenen sauren Gruppen haben eine Doppelfunktion, indem sie einerseits die ionische Bindung der hydrophilen bioaktiven Schicht an die basischen Gruppen des Polyalkylenimin-Primers bzw. des mit Ammoniak-Plasma behandelten polymeren Substrats bewirken und andererseits eine biologische Wirkung entfalten, gegebenenfalls nach Neutralisation, z.B. mit Natronlauge.

Die Beschichtungspolymeren können Homo- oder Copolymere von hydrophilen Vinylmonomeren oder Copolymere von hydrophilen und hydrophoben Vinylmonomeren sein. Geeignete hydrophile und hydrophobe Vinylmonomere sind z.B. die zuvor unter Ziffer 4.1 aufgeführten Monomeren. Ein Beschichtungspolymer gilt als hydrophil im Sinne der Erfindung, wenn sein Kontaktwinkel bei 25°C nach P.J.Goods et al. <35°, vorteilhaft <25° ist. Dieser Bedingung genügen Beschichtungs(co)polymere, die ausschließlich aus hydrophilen Monomeren aufgebaut sind, in jedem Fall. Wenn ein Beschichtungscopolymer hydrophobe Vinylmonomere enthält, darf deren Anteil nur so hoch sein daß der Kontaktwinkel der genannten Bedingung entspricht. Die Beschichtungs(co)polymeren werden nach üblichen Verfahren hergestellt. z.B. durch radikalisch initiierte Lösungs- oder Emulsionspolymerisation in wäßrigem Medium. Vielfach sind die erfindungsgemäß zu verwendenden Beschichtungspolymere so hydrophil, daß ein Kontaktwinkel nicht mehr meßbar ist, weil der Wassertropfen auf der Oberfläche spreitet.

Von den geeignete hydrophilen bioaktiven Beschichtungs(co)polymeren seien die folgenden beispielhaft genannt:
(i) Poly(meth)acrylsäure sowie hydrophile Copolymere der Acrylsäure oder Methacrylsäure mit anderen sauren oder neutralen, hydrophilen oder hydrophoben Comonomeren, wobei die Carboxylgruppen schon in den Monomeren zum Teil oder später im Polymer zum Teil oder vollständig neutralisiert werden können. Diese Polymeren enthalten keine Sulfonsäure und/oder Sulfonatgruppen. Geeignete saure oder neutrale, hydrophile oder hydrophobe Comonomere sind z.B. die zuvor unter Ziffer 4.1 beschriebenen.
(ii) Andere geeignete hydrophile bioaktive Beschichtungspolymere sind Copolymere aus olefinisch ungesättigten Carbonsäuren oder deren Anhydriden und olefinisch ungesättigten Sulfonsäuren sowie gegebenenfalls anderen neutralen und hydrophilen oder hydrophoben Comonomeren. Auch hier gilt, daß die sauren Gruppen zum Teil schon in den Monomeren oder später im Polymer zum Teil oder vollständig neutralisiert werden können. Von den olefinisch ungesättigten Carbonsäuren seien Acrylsäure, Methacrylsäure, Crotonsäure, Maleinsäure und Maleinsäureanhydrid (das unter den Reaktionsbedingungen hydrolysiert), Vinylsalicylsäure, Itaconsäure, Vinylessigsäure, Zimtsäure, 4-Vinylbenzoesäure, 2-Vinylbenzoesäure, Sorbinsäure, Kaffeesäure, Methylmaleinsäure, Isocrotonsäure, Fumarsäure, Methylfumarsäure, Dimethylfumarsäure, Dihydroxymaleinsäure und Allylessigsäure sowie deren Natriumsalze genannt. Geeignete olefinisch ungesättigte Sulfonsäuren sind z.B. Vinylsulfonsäure, 2- und 4-Styrolsulfonsäure, Allylschwefelsäure, Methallylschwefelsäure, Methallylsulfonsäure, Vinyltoluolsulfonsäure sowie deren Natriumsalze. Geeignete Copolymere dieser Art sind in den deutschen Patentanmeldungen 197 00 076.2 (O.Z. 5139), 197 00 077.0 (O.Z. 5140), 197 00 078.9 (O.Z. 5141), 197 23 132.2 (O.Z. 5202) und 197 23 131.4 (O.Z. 5203) beschrieben.
(iii) Weitere geeignete Beschichtungspolymere sind die heparinartigen Copolymeren, die in der deutschen Patentanmeldung 192 20 369.8 (O.Z. 5195) beschrieben sind. Sie enthalten die Repetiereinheiten 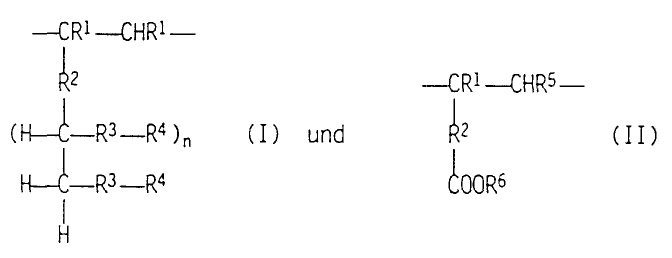 in denen R¹ jeweils unabhängig Wasserstoff oder den Methylrest; R² einen zweiwertigen organischen Rest, beispielsweise einen aliphatischen, cycloaliphatischen oder aromatischen Rest mit bis zu 10 Kohlenstoffatomen (wie o-Phenylen, m-Phenylen und p-Phenylen), oder eine C-C-Einfachbindung; R³ -O- oder -NH-; R⁴ Wasserstoff oder den Rest -SO₃⁻Na⁺; R⁵ Wasserstoff, den Methylrest oder den Rest -R²-COOR⁶; R⁶ Wasserstoff oder Na und n = 4 oder 5 bedeuten; mit der Maßgabe, daß mindestens einer der Substituenten R⁴ ein Rest -SO₃⁻Na⁺ ist. Vorteilhaft steht zumindest in einem Teil der Gruppen R⁶ für Wasserstoff. Die Bausteine (I) gehen auf Vinylmonomere der allgemeinen Formel III 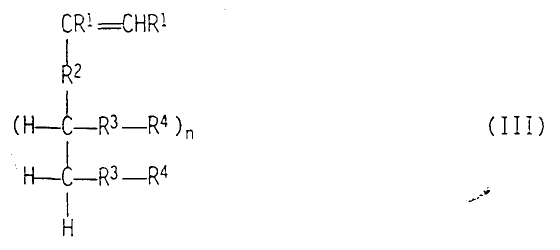 zurück, in der R¹, R², R³, R⁴ und n die angegebenen Bedeutungen haben. Geeignete Vinylmonomere I sind z.B. O-sulfatiertes 1-Hydroxy-1-desoxy-1-(4-vinylphenyl)-D-gluco(D-manno)-pentitol (Ia) und N-und O-sulfatiertes 1-Amino-1-desoxy-1-(4-vinylphenyl)-D-gluco(D-manno)-pentitol (Ib) bzw. deren Natriumsalze. Beide Monomere werden in einer mehrstufigen, von D-Glucono-1,5-lacton ausgehenden Synthese gewonnen, wie in der genannten deutschen Patentanmeldung beschrieben. Dort wird auch die Herstellung von 4-Vinylbenzoesäure beschrieben, die bzw. deren Natriumsalz ein geeignetes Monomer II ist.
(iv) Weitere geeignete Beschichtungspolymere sind die in der deutschen Patentanmeldung 198 01 040.0 (O.Z. 5281) offenbarten Homo- oder oder Copolymere, die Repetiereinheiten der Formel IV 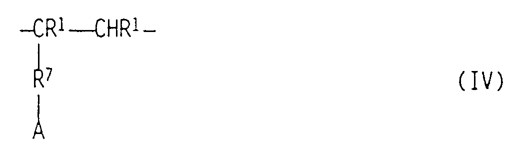 enthalten, in der R¹ die für die Formel I angegebene Bedeutung hat, R⁷ ein Brückenglied und A einen sulfatierten Polyol-, Polyamin- oder (Poly)amin-(poly)ol-Rest bedeutet, der gegebenenfalls eine oder mehrere acetalisierte oder aminalisierte Carbonylfunktionen enthält. Das Brückenglied R⁷ kann anorganischer oder organischer Natur sein und steht bevorzugt für O, S, SO, SO₂ oder NR', wobei R' einen Kohlenwasserstoffrest mit 1 bis 12 Kohlenstoffatomen bezeichnet, für einen zweiwertigen organischen Rest, insbesondere für einen aliphatischen, cycloaliphatischen oder aromatischen Kohlenwasserstoffrest mit bis zu 10 Kohlenstoffatomen, eine Carbonesterbrücke -O-CO-, eine Carbonamidbrücke -NR' -CO- oder eine Urethanbrücke -O-CO-NR'-, wobei R' jeweils die angegebenen Bedeutung hat, oder für eine C-C-Einfachbindung.

Bevorzugte Repetiereinheiten IV mit acetalisierter oder aminalisierter Carbonylfunktion entsprechen der Formel V 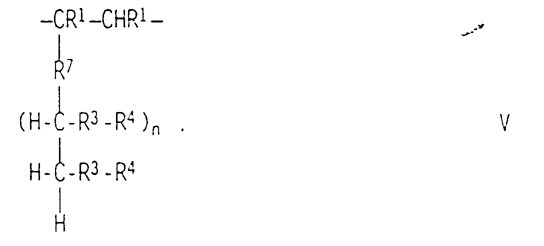 in der
R¹, R³, R⁴, R⁷ und n die für die Formel I angegebenen Bedeutungen haben; mit der Maßgabe, daß
   (1) mindestens einmal, vorteilhaft ein- oder zweimal pro Molekül an demselben Kohlenstoffatom stehende Substituenten H und -R³-R⁴ zusammen mit diesem Kohlenstoffatom eine Carbonylfunktion C=O bilden, die durch eine Hydroxyl- bzw. Aminofunktion in 3-Stellung in bezug auf die Carbonylfunktion unter Bildung eines Tetrahydrofuran- bzw. Pyrrolidinringes oder durch eine Hydroxyl- bzw. Aminofunktion in 4-Stellung in bezug auf die Carbonylfunktion unter Bildung eines Pyran- bzw. Pentamethyleniminringes intramolekular acetalisiert bzw. aminalisiert ist, und daß (2) mindestens einer der Substituenten R⁴ ein Rest -SO₃⁻Na⁺ ist.

Den Repetiereinheiten V im (Co)polymer entsprechen die Monomeren der Formel Va 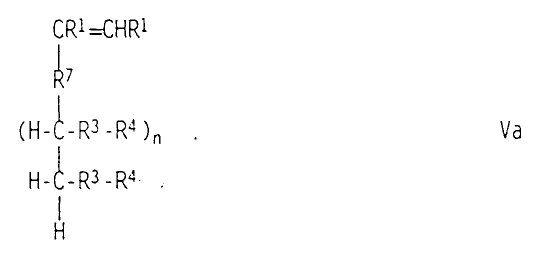 in der
R¹, R³, R⁴, R⁷ und n die für die Formel V angegebenen Bedeutungen einschließlich der Maßgabebedingungen haben.

Geeignete Monomere Va sind z.B. die O-sulfatierte 1-(4-Vinylphenyl)-D-manno(D-gluco)-hexulo-2,6-pyranose (VaA) sowie die O-sulfatierte 6-(4-Vinylphenyl)-D-glycero(L-glycero)-α-D-galactopyranose (VaB). Die Herstellung dieser Monomeren ist ebenfalls in der deutschen Patentanmeldung 198 01 040.0 (O.Z. 5281) beschrieben.

Zur Beschichtung der mit dem Haftvermittler vorbehandelten hydrophilen oder hydrophilierten polymeren Substrate läßt man die Beschichtungspolymeren zweckmäßig in 1- bis 20-gewichtsprozentiger, vorzugsweise wäßriger Losung 0,1 bis 10 Minuten auf die polymeren Substrate einwirken, in der Regel bei 25 bis 50°C. Danach werden die Substrate getrocknet. Noch vorhandene saure Gruppen können oberflächig durch Behandlung mit einer Base, z.B. mit Natronlage, ganz oder teilweise in Carboxylat- und/oder Sulfonatgruppen überführt werden. Der Schichtaufbau aus Primer und hydrophilem bioaktiven Polymer haftet auch dann fest auf dem polymeren Substrat und läßt sich z.B. nicht durch Einwirkung von 60°C heißem Wasser ablösen.

### 4.5 Wiederholte Beschichtung

Für manche Anwendungen ist es empfehlenswert den Beschichtungsvorgang, gegebenenfalls einschließlich der Behandlung mit dem Polyalkylenimin bzw. mit Ammoniak-Plasma, zu wiederholen, um eine vollständige Bedeckung des hydrophilen oder hydrophilierten polymeren Substrats mit der hydrophilen bioaktiven Schicht zu erreichen. Bei Behandlung mit einem Polyalkylenimin sind zweckmäßig in dem zuerst aufgebrachten hydrophilen bioaktiven Beschichtungsmittel Carboxyl- und gegebenenfalls Sulfonsäuregruppen vorhanden, damit der Haftvermittler ionisch gebunden werden kann. Die sauren Gruppen des Beschichtungsmittels dürfen also vor dem Aufbringen auf das Substrat mit dem Haftvermittler allenfalls zum Teil mit einer Base, wie Natronlauge, neutralisiert worden sein. Nach dem Aufbringen können sie dann, wie zuvor erwähnt, vollständig neutralisiert werden. Im übrigen gelten für die wiederholten Beschichtungsvorgänge (Behandlung mit Polyalkylenimin bzw. Ammoniak-Plasma und Aufbringen des Beschichtungspolymers) sinngemäß die zuvor gegebenen Erläuterungen.

### 4.6 Bioaktive Eigenschaften der beschichteten Substrate

Die erfindungsgemäß beschichteten Substrate sind hydrophil und bakterienabweisend. Beschichtungen aus den Beschichtungspolymeren 4.4 (iii) und 4.4(iv) zeichnen sich zudem im befeuchteten Zustand durch einen besonders niedrigen Reibungskoeffizienten aus. In Beschichtungspolymeren, die Carboxyl- und/oder Carboxylatgruppen sowie Sulfonsäure und/oder Sulfonatgruppen aufweisen, kann das molare Verhältnis dieser Gruppen in weiten Grenzen schwanken. Neben dem hydrophilen und bakterienabweisenden Charakter zeigen die bioaktiv beschichteten polymeren Substrate ausgeprägte zellproliferationsinhibierende Eigenschaften, wenn das genannte molare Verhältnis 0,2 bis 3 und insbesondere 0,4 bis 2 beträgt. Die beschichteten Oberflächen zeigen in bemerkenswerter Weise bakterienabweisende, aber zellproliferationsfördernde Eigenschaften, wenn das besagte molare Verhältnis 2 bis 10, vorteilhaft 3 bis 10 und insbesondere 3 bis 5 beträgt. Als zellproliferationsfördernd wird eine Beschichtung dann angesehen, wenn die Haftung und Vermehrung von Säugetierzellen durch die Beschichtung im Vergleich zu der unbeschichteten Oberfläche verbessert oder jedenfalls weniger stark beeinträchtigt wird.

Beschichtungspolymere 4.4(iii) und 4.4(iv) zeichnen sich besonders durch heparinanaloge Wirksamkeit aus, die in den deutschen Patentanmeldung 192 20 369.8 (O.Z. 5195) und 198 01 040.0 (O.Z. 5281) näher beschrieben wird.

### 4.7 Erzeugnisse und deren Verwendung

Erzeugnisse mit einer nach dem Verfahren der Erfindung beschichteten Oberfläche eignen sich zur Verwendung für technische, medizintechnische, hygienische oder biotechnische Zwecke, wie sie zuvor erwähnt wurden. Wenn es bei der Verwendung der nach dem Verfahren der Erfindung hydrophil beschichteten Substrate auf Monomerenfreiheit ankommt, ist es zweckmäßig, die Restmonomeren aus der polymeren hydrophilen Beschichtung zu extrahieren. Dies kann mit Wasser und anschließend mit einem organischen Lösungsmittel geschehen, z.B. mit Kohlenwasserstoffen, wie Hexan oder Cyclohexan, und/oder mit einem Alkanol mit 1 bis 4 Kohlenstoffatomen, wie Ethanol und n-Propanol. Gut geeignet für den zweiten Extraktionsschritt ist z.B. ein Gemisch aus n-Hexan und Ethanol mit 65 bis 85 Volumenprozent n-Hexan.

Von den Erzeugnissen zur technischen, biotechnischen, hygienischen oder medizinischen Verwendung seien z.B. Katheter, Schläuche, Stents, Membranen, Blutbeutel, Wundverbände, Oxygenatoren, elastische Handschuhe für medizinische Zwecke und Kondome genannt.

### 5. Beispiele

### Materialien und Verfahrensweisen

**Tabelle 1**

| **Eingesetzte Substratfolien (F) und -schläuche (Sch)** | | | |
|---|---|---|---|
| Folie/Schlauch Nr. | Kunststoff | Name, Quelle | Herstellung |
| F 1 | Polyamid 12 | VESTAMID^{(R)}, HÜLS AG | Extrudieren |
| Sch 2 | Polyurethan | TECOFLEX^{(R)}, THERMEDIX GmbH | Extrudieren |
| Sch 3 | Polyetheresteramid | VESTAMID^{(R)}, HÜLS AG | Extrudieren |
| F 4 | Polyurethan | PELLETHANE^{(R)} 2363-A DOW CHEMICAL COMPANY | Extrudieren |
| F 5 | Polyethylen | VESATOLEN^{(R)} A, VESTOLEN GmbH | Extrudieren |
| F 6 | Polypropylen | VESTOLEN^{(R)} P, VESTOLEN GmbH | Extrudieren |
| F 7 | Polyorganosiloxan | NG 37-52, Silicon GmbH, Nünchritz | Rakeln |
| F 8 | Polyvinylchlorid | VESTOLIT^{(R)}+Diethylhexylphthalat, VESTOLIT GmbH | Brabandern |
| F 9 | PTFE | HOSTAFLON^{(R)}, HOECHST AG | Extrudieren |
| F 10 | Polystyrol | VESTYRON^{(R)}, HÜLS AG | Pressen |
| Sch 11 | Polyethylen | VESTOLEN^{(R)} A, VESTOLEN GmbH | Extrudieren |
| Sch 12 | Polyurethan | TECOFLEX^{(R)}, THERMEDIX GmbH | Extrudieren |
| Sch 13 | Polyurethan | PELLETHANE^{(R)} 2363A, DOW CHEMICAL CO. | Extrudieren |
| Sch 14 | Polyetheresteramid | PEBAX^{R)} 5533, ATOCHEM S.A. | Extrudieren |
| Sch 15 | Polyvinylchlorid | VESTOLIT^{(R)}+Diethylhexylphthalat, VESTOLIT GmbH | Extrudieren |

Die Folien bzw. Schläuche wurden überwiegend zunächst aktiviert. d.h. hydrophiliert und, soweit die Hydrophilierung mit Ammoniakplasma erfolgte, zugleich erfindungsgemäß behandelt. Die Aktivierung erfolgte wahlweise nach den in Tabelle 2 angegebenen Verfahren und Bedingungen.

**Tabelle 2**

| **Aktivierungsmethoden** | | |
|---|---|---|
| Aktivierungskennzeichen | Aktivierungsverfahren | Bedingungen |
| A 1 | UV-Excimer-Strahlen (172 nm) | 1 s - 20 min, 1 mbar, 4 cm Abstand |
| A 2 | Mikrowellenplasma (Argon) | 1 s - 30 min, 1 mbar, |
| A 3 | Hochfrequenzplasma (Argon) | 1 s - 30 min, 6 mbar |
| A 4 | Corona | 0.1s - 60s, 2 mm Abstand |
| A 5 | Beflammen | CH₄:Luft=1:10;4cm Abstand |
| A 6 | NaOH-Lösung | 1%; 5 min; 60°C |
| A 7 | Mikrowellenplasma (NH₃) | 10 sec - 3 min; 35 - 150Pa |
| A 8 | Hochfrequenzplasma (NH₃) | 10 sec - 3 min; 35 - 150Pa |

**Tabelle 3**

| **Verwendete Polyalkylenimine** | |
|---|---|
| Bezeichnung | Produktmerkmale |
| P 1 | Polyethylenimin mit einem mittleren Molekulargewicht von 750.000 (ermittelt nach der Lichtstreuungs-Methode) in Form einer etwa 50 gewichtsprozentigen wäßrigen Lösung mit einem pH in 1 %-iger Lösung von 10 - 12 (LUPASOL^{(R)}P der BASF AG), mit Wasser verdünnt auf 2.5 Gew.-% Feststoff |
| P 2 | Polyethylenimin mit einem mittleren Molekulargewicht von 25.000 (ermittelt nach der Lichtstreuungs-Methode) mit einem Feststoffgehalt von 99%, einem pH in 1 %-iger Lösung von 10 - 12 (LUPASOL^{(R)}WF der BASF AG), mit Wasser verdünnt auf 1 Gew.-% Feststoff (P2a) bzw. 5 Gew.-% Feststoff (P2b) verdünnt. |

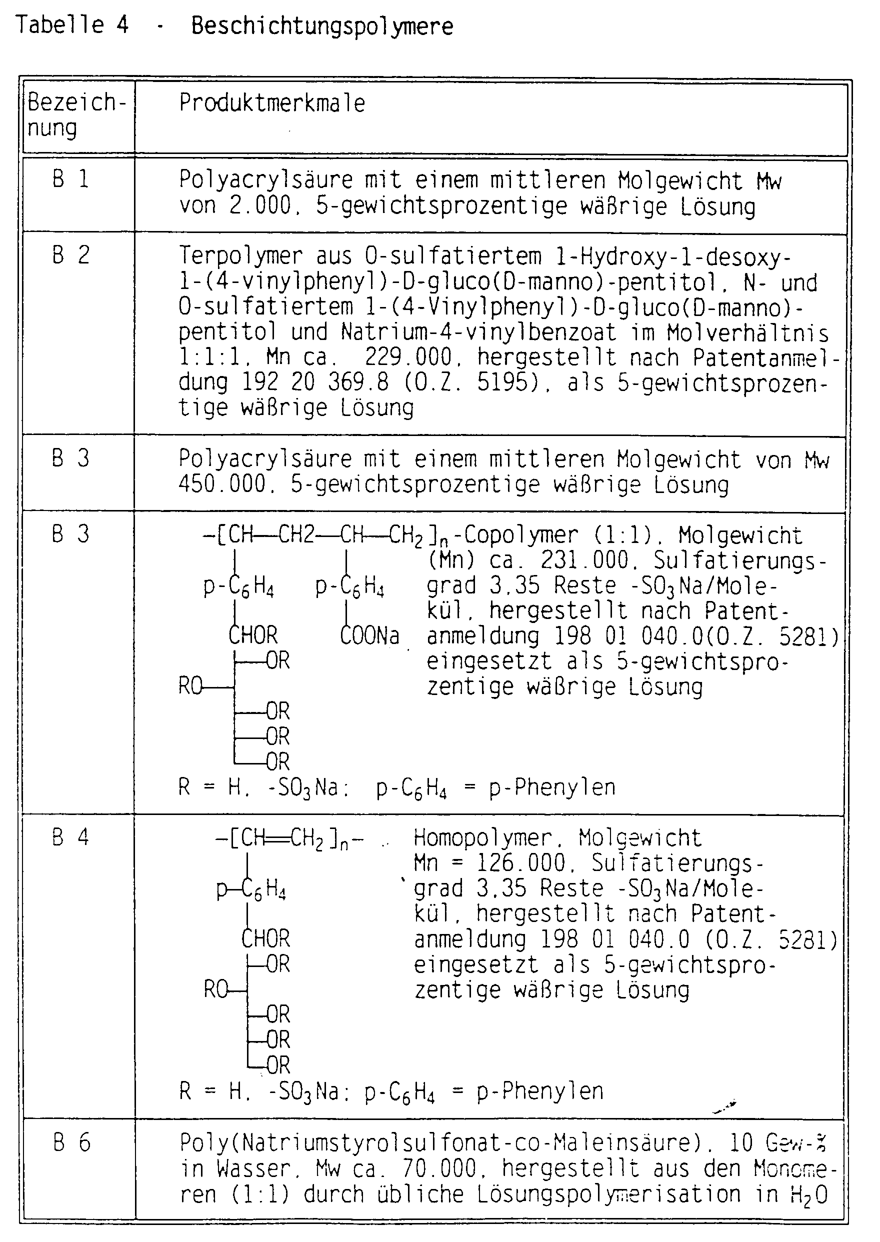

### Behandlung mit Polyethylenimin

Die Folien bzw. Schläuche wurden in die Polyethylenimin-Lösung von 25°C getaucht, innerhalb von 20 sec langsam herausgezogen und 5 min bei 60°C getrocknet, in die Lösung des Beschichtungspolymers von 25°C getaucht, innerhalb von 20 sec langsam herausgezogen und wiederum 5 min bei 60°C getrocknet. Dieser Zyklus wurden gegebenenfalls mehrmals wiederholt, wie in Tabelle 5 angegeben.

### Behandlung mit Ammoniak-Plasma

Die zu behandelnden Proben werden entweder auf einen Glasträger aufgeklebt oder an einer Glasvorrichtung montiert, so daß sie bei Anlegen von Unterdruck ihre Position in der Plasmakammer nicht verändern. Anschließend wird der Druck auf 5 bis 20 Pa vermindert und Ammoniak so zudosiert und abgesaugt, daß sich ein Druck von 10 bis 500 Pa, vorzugsweise 30 bis 180 Pa, einstellt und aufrechterhalten wird. Nach 20 sec Homogenisierungszeit für das Gas wird das Plasma gezündet. Die optimale Leistung pro Flächeneinheit des Substrats hängt von der Größe der Plasmakammer ab und läßt sich durch Vorversuche unschwer ermitteln. Die Behandlungsdauer kann 1 sec bis 30 min betragen, je nach Substrat und gewünschtem Ausmaß der Erzeugung von basischen Gruppen. Anschließend wird die Plasmakammer etwa 1 min mit Luft gespült. Gegebenfalls wird erneut Vakuum angelegt, um letzte Spuren Ammoniakgas zu entfernen.

### Meß- und Prüfmethoden

### Molekulargewichte

Alle Molekulargewichte Mn wurden durch Membranosmometrie, alle Molekulargewichte Mw nach der Lichtstreuungsmethode bestimmt.

### Bestimmung von COOH und S auf der Beschichtung

Mittels ESCA wurde der gegenüber der unbehandelten Probe erhöhte Sauerstoffgehalt auf der Oberfläche bestimmt, der auf die Carboxyl- und/oder Carboxylat, Sulfonsäure- und oder Sulfonat- und/oder sauren Schwefelsäureester und/oder -sulfat-Gruppen zurückgeht. Die schwefelhaltigen Gruppen des Beschichtungspolymers wurden durch Messung des Schwefels als Oberflächenelement nachgewiesen.

### Bestimmung des Kontaktwinkels

Ein Maß für die Hydrophilie einer Oberfläche ist die Veränderung des Kontaktwinkels eines Wassertropfens, der auf die Oberfläche aufgesetzt wird. Ein solches Verfahren wurde von R.J.Good et al., Techniques of Measuring Contact Angles in Surface and Colloid Science (Hrsg. R.J.Good), Vol. 11, Plenum Press New York, N.Y., 1979 beschrieben. In den Beispielen wurden die Kontaktwinkel nach dieser Vorschrift bei 25°C gemessen. Bei stark hydrophilen Substraten läßt sich der Kontaktwinkel nicht mehr messen, weil sich die Flüssigkeit in dünner Schicht auf dem Substrat verteilt. In Tabelle 6 wird das als Spreitung bezeichnet.

### Bestimmung des Reibungskoeffizienten

Der Reibungskoeffizient µ wurde nach der Methode der schiefen Ebene bestimmt. Hierzu wird die beschichtete Substratfolie auf eine runde Metallscheibe aufgeklebt und in Wasser bzw. einer 0.9 Gew.-% NaCl-Lösung über verschiedene Zeiten gelagert (in Tabelle 4 "Wässern min") Anschließend wird die Metallscheibe mit der Folie nach unten auf eine mit dem unbeschichteten Polymer überzogene ebene Platte gesetzt, die mit destilliertem Wasser befeuchtet war. Der Winkel der Platte zur Horizontalen wurde langsam vergrößert. Der Winkel, bei dem die Metallscheibe zu gleiten begann, wurde festgehalten. Sein Tangens ist der Reibungskoeffizient µ.

### Bestimmung der primären Bakterienadhäsion unter statischen Bedingungen

Eine Über-Nacht-Kultur des Bakterienstammes Klebsiella pneumoniae in Hefeextrakt-Pepton-Glukose-Nährmedium (1% + 1% + 1%) wird abzentrifugiert und in Phosphat-gepufferter Saline (=PBS; 0,05m KH₂PO₄, pH 7,2 + 0,9 % NaCl) wieder aufgenommen. Man verdünnt mit PBS-Puffer auf ein Zellkonzentration von 10⁸ Zellen/ml. Die suspendierten Bakterien werden mit dem zu untersuchenden Folienstück für 3h in Berührung gebracht. Dazu werden doppelseitig beschichtete kreisförmige Folienstücke mit einem Durchmesser von 1.6 cm (=4.02 cm²) auf eine Präpariernadel gesteckt und mit der Zellsuspension geschüttelt. Einseitig beschichtete Folien werden in Form einer runden, ebenen Scheibe von 4.5 cm Durchmesser und mit einer Stützmembran aus 2-3 cm dickem Weich-PVC in eine Membranfilterapparatur eingespannt. Auf die nach oben zeigende Seite mit der zu prüfenden Beschichtung wird die Zellsuspension aufgegeben und 3h geschüttelt. Die Membranfilterapparatur muß dicht sein, d.h. es darf keine Zellsuspension durch undichte Zellen ausfließen.

Nach Ablauf der Kontaktzeit wird die Bakteriensuspension mit einer Wasserstrahlpumpe abgesaugt, und die Folienstücke werden zum Waschen mit 20 ml steriler PBS-Lösung in einem 100 ml Becherglas 2 min geschüttelt. Das Folienstück wird nochmals in sterile PBS-Lösung eingetaucht und dann in 10 ml erhitztem TRIS/EDTA (0,1M Trishydroxyethylaminomethan, 4 mM Ethylendiamintetraessigsäure, mit HCl auf pH 7,8 eingestellt) für 2 min im siedenden Wasserbad extrahiert.

Mit der Extraktionslösung werden kleine Eppendorf-Cups befüllt und sofort bis zur Biolumineszenz-Bestimmung des extrahierten Adenosintriphosphats (ATP) bei -20°C eingefroren. Die Bestimmung wird wie folgt ausgeführt: In ein transparentes Röhrchen aus Polycarbonat wird 100 µl Reagentienmix (Biolumineszenz-Test CLS II, Fa. BOEHRINGER MANNHEIM GmbH) gegeben, und über einen Zeitraum von 10 sec werden in einem Lichtimpuls-Meßgerät LUMAT LB9501 (Laboratorien Prof. Berthold GmbH, 75323 Bad Wildbad, Deutschland) die Lichtimpulse integriert. Dann wird eine 100 µl Probe zugegeben und erneut gemessen. Die relativen Lichteinheiten (RLU) werden durch Subtraktion der Lichtimpulse im Reagentienmix von der Anzahl der gemessenen Lichtimpulse im kompletten Ansatz erhalten. Dieser Wert steht in Relation zu der Anzahl der an der Folie adhärierten Bakterien. Der Umrechnungsfaktor zwischen dem RLU-Wert und der Bakterienzahl wird bestimmt, indem ein Aliquot von 0.1 ml der Bakteriensuspension mit 10⁸ Zellen/ml in 10 ml heißem TRIS/EDTA extrahiert und dann der ATP-Gehalt bestimmt wird. In Tabelle 6 wurde die Bakterienadhäsion auf den unbehandelten Substraten gleich 100% gesetzt (= 0% Verminderung der Bakterienadhäsion).

In entsprechender Weise und mit ähnlichen Ergebnissen verlaufen Versuche mit anderen Bakterienstämmen, wie Staphylococcus aureus, Staphylococcus epidermidis, Streptococcus pyogenes, Pseudomones aeroginosa und Escherichia coli.

### Versuchsergebnisse

### Beispiele 1 bis 30

Die Versuchsbedingungen und die erhaltenen Ergebnisse gehen aus der folgenden Tabelle 5 hervor.

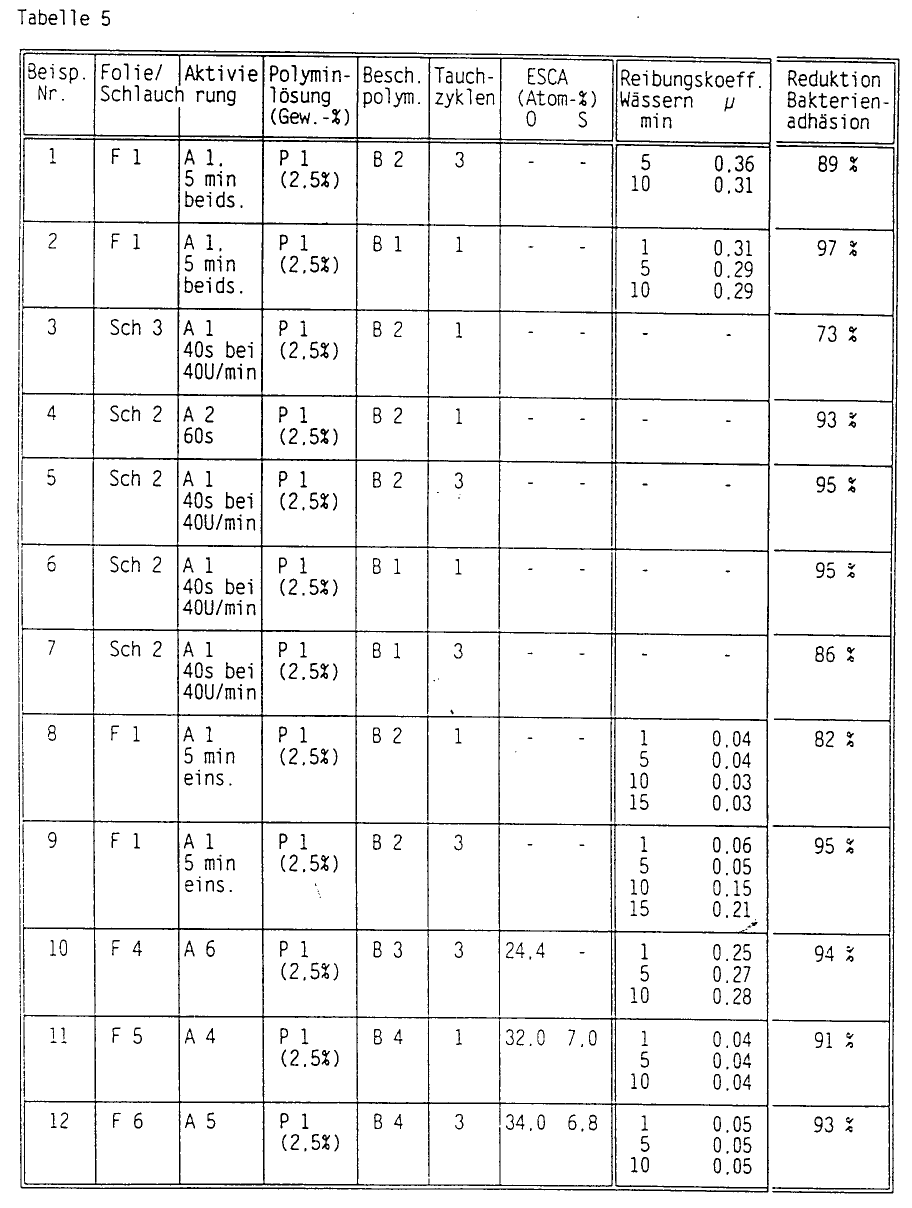

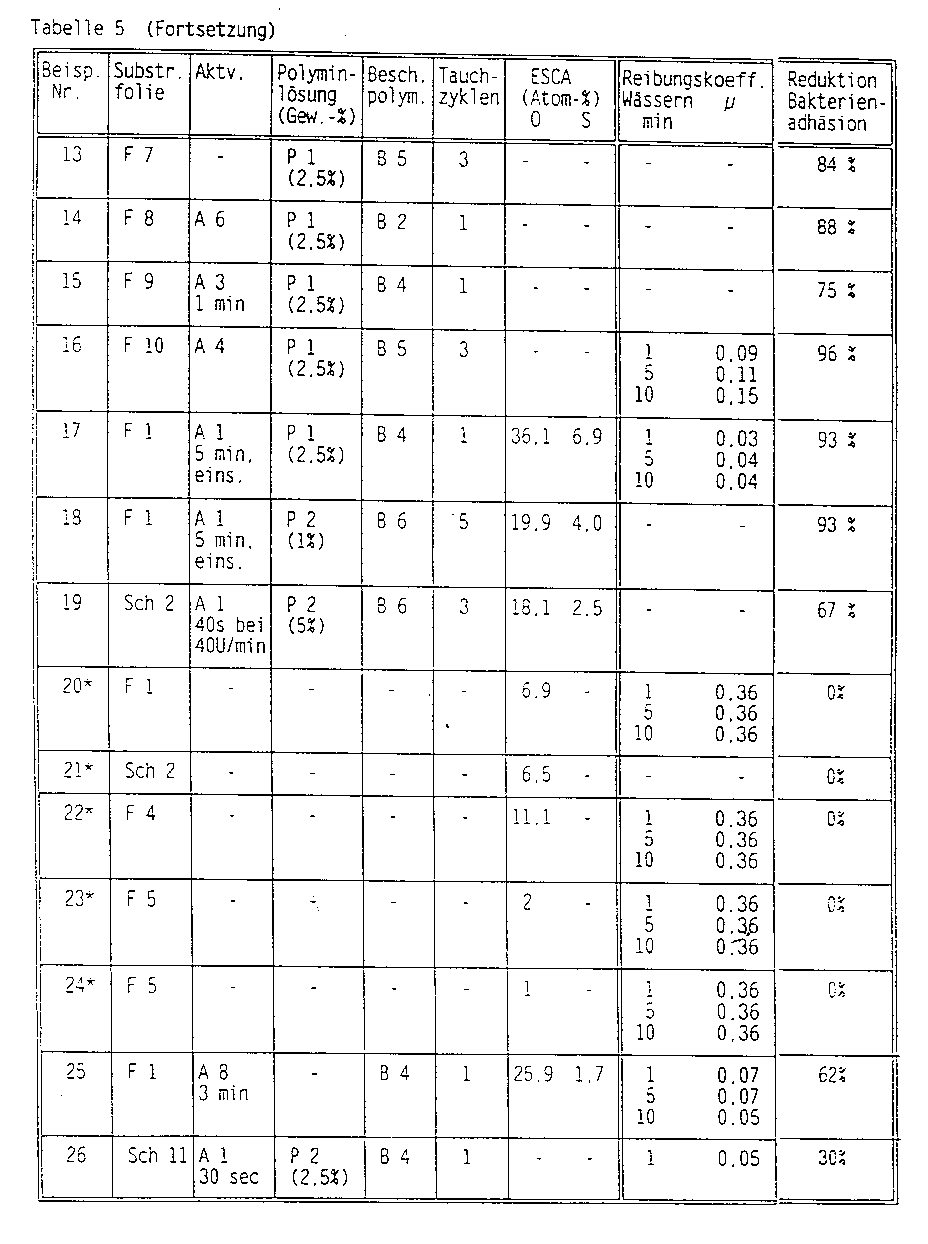

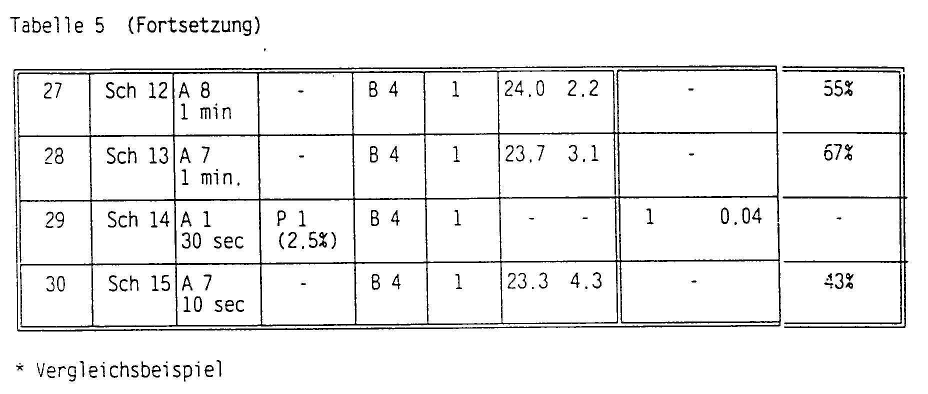

Bei den Beispielen 1 bis 19 und 25 bis 30 konnte ein Kontaktwinkel infolge Spreitung nicht gemessen werden.

### Beispiel 31

Eine Polyamid 12-Folie (L2101F der Hüls AG) wurde 5 min bei 1 Torr mit einer 172 nm-Excimerlampe bestrahlt, anschließend in eine 2,5 gew. -prozentige Lösung von Lupasol P (BASF Aktiengesellschaft) (P1 nach Tabelle 3) getaucht und 5 min bei 60°C getrocknet. Die getrocknete Folie wird 20 sec in eine 5-gewichtsprozentige Lösung des Beschichtungspolymers B 5 getaucht und 72 h bei 60°C getrocknet. Der Reibungskoeffizient µ lag bei 0,07 bis 0,1, im Vergleich zu 0.36 für das unbeschichtete Material. Ein Kontaktwinkel konnte nicht gemessen werden, da der Tropfen spreitete.

### Beispiel 32

Eine Folie aus einem thermoplastischen Elastomer wurde zur Hydrophilierung 3 mal 10 sec in Aceton getaucht und bei Raumtemperatur getrocknet, anschließend 30 sec in eine 2,5 gew.-prozentige wäßrige Lösung von Lupasol P (BASF AG) (P 1 nach Tabelle 3) getaucht, 5 min bei Raumtemperatur und 30 min im Trockenschrank bei 60°C getrocknet. Die getrocknete Folie wurde dann 30 sec in die Lösung eines Zuckerpolymers der Formel 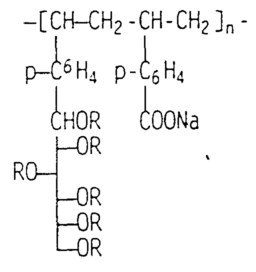
R = H, -SO₃Na; p-C₆H₄ = p-Phenylen
(Copolymer 1:1, Molgewicht Mn = 570.000 Sulfatierungsgrad 3.09 Reste -SO₃Na/Molekül, hergestellt nach Patentanmeldung 195 20 369.8 (O.Z. 5195), eingesetzt als 5-gewichtsprozentige wäßrige Lösung) getaucht und 5 min bei Raumtemperatur sowie 30 min im Trockenschrank bei 60°C getrocknet. Der Reibungskoeffizient µ lag bei 0,02. Ein Kontaktwinkel konnte nicht gemessen werden, da der Tropfen spreitete. Ein Abrieb der Beschichtung wurde bei einem Fingertest erst nach 58-maligem Überstreichen spürbar.

### Beispiel 33

Man verfuhr wie in Beispiel 32, anstelle der Acetonwäsche wurde die Folie jedoch 10 sec bei 1 Torr mit einem 172-nm Excimerstrahler bestrahlt. Die Beschichtung konnte bei dem Fingertest erst nach 120-maligem Überstreichen abgerieben werden.

## Patentansprüche

1. Verfahren zur hydrophilen und bioaktiven Beschichtung von polymeren Substraten, dadurch gekennzeichnet, daß man ein hydrophiles oder hydrophiliertes polymeres Substrat mit einem Polyalkylenimin als Haftvermittler behandelt und auf das behandelte Substrat ein (a) Carboxyl- und/oder Carboxylatgruppen und/oder (b) Sulfonsäure- und/oder Sulfonatgruppen und/oder (c) saure Schwefelsäureester- und/oder -sulfatgruppen enthaltendes hydrophiles bioaktives Beschichtungspolymer aufbringt.

2. Verfahren nach Anspruch 1, dadurch gekennzeichnet, daß das hydrophile oder hydrophilierte polymere Substrat saure Gruppen enthält.

3. Verfahren nach Anspruch 1 oder 2, dadurch gekennzeichnet, daß das hydrophile oder hydrophilierte Substrat einen Kontaktwinkel bei 25°C, gemessen nach der Methode von R.J.Goods et al., von <40° zeigen.

4. Verfahren nach Anspruch 1 oder 2, dadurch gekennzeichnet, daß das hydrophile oder hydrophilierte Substrat einen Kontaktwinkel bei 25°, gemessen nach der Methode von R.J.Goods et al., von <30° zeigen.

5. Verfahren nach einem der Ansprüche 1 bis 4, dadurch gekennzeichnet, daß das Polyalkylenimin aus einer 1- bis 20-gewichtsprozentigen wäßrigen Lösung auf das polymere Substrat aufgebracht wird.

6. Verfahren nach einem der Ansprüche 1 bis 5, dadurch gekennzeichnet, daß das Polyalkylenimin Polyethylenimin ist.

7. Verfahren nach Anspruch 6, dadurch gekennzeichnet, daß das Polyethylenimin ein zahlendurchschnittliches Molekulargewicht von 10.000 bis 2.000.000 hat.

8. Verfahren nach Anspruch 1, dadurch gekennzeichnet, daß das gegebenenfalls auch nicht hydrophile oder hydrophilierte polymere Substrat statt mit einem Polyalkylenimin mit einem Ammoniak-Plasma behandelt wird.

9. Verfahren nach einem der Ansprüche 1 bis 8, dadurch gekennzeichnet, daß das Beschichtungspolymer Carboxyl-, Sulfonsäure- und bzw. oder saure Schwefelsäureester-Gruppen enthält.

10. Verfahren nach einem der Ansprüche 1 bis 8, dadurch gekennzeichnet, daß das Beschichtungspolymer haftend aufgebracht wird, indem man auf das mit dem Polyalkylenimin oder dem Ammoniak-Plasma behandelte polymere Substrat eine Lösung eines hydrophilen und bioaktiven Carboxyl-, Sulfonsäure- oder saure Schwefelsäureester-Gruppen enthaltenden Beschichtungspolymers einwirken läßt.

11. Verfahren nach Anspruch 10, dadurch gekennzeichnet, daß das Beschichtungspolymer Polyacrylsäure oder ein Copolymer der Acrylsäure oder Methacrylsäure oder Maleinsäure mit anderen sauren oder neutralen, hydrophilen oder hydrophoben Comonomer ist.

12. Verfahren nach Anspruch 10, dadurch gekennzeichnet, daß das Beschichtungspolymer ein Copolymer aus olefinisch ungesättigten Carbonsäuren oder deren Anhydriden und olefinisch ungesättigten Sulfonsäuren sowie gegebenenfalls anderen neutralen, hydrophilen oder hydrophoben Comonomeren ist.

13. Verfahren nach Anspruch 10, dadurch gekennzeichnet, daß das Beschichtungspolymer ein Copolymer ist, das die Repetiereinheiten 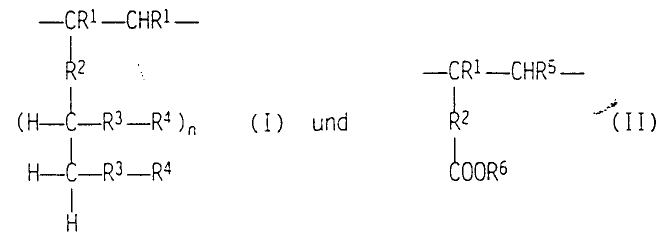 enthält, in denen R¹ Wasserstoff oder den Methylrest; R² einen zweiwertigen organischen Rest oder eine C-C-Einfachbindung: R³ -O- oder -NH-; R⁴ Wasserstoff oder den Rest -SO₃⁻Na⁺; R⁵ Wasserstoff, den Methylrest oder den Rest -R²-COOR⁶; R⁶ Wasserstoff oder Na und n = 4 oder 5 bedeuten; mit der Maßgabe, daß mindestens einer der Substituenten R⁴ ein Rest -SO₃⁻Na⁺ ist und daß zumindest ein Teil der Gruppen -COOR⁶ Carboxylgruppen sind.

14. Verfahren nach Anspruch 10, dadurch gekennzeichnet, daß das Beschichtungspolymer ein Homo- oder Copolymer ist, das Repetiereinheiten der Formel IV 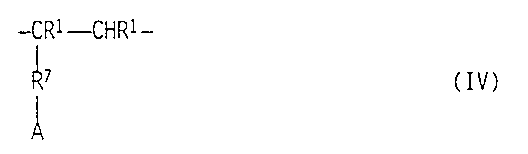 enthält, in der R¹ die für die Formel I angegebene Bedeutung hat. R⁷ ein Brückenglied und A einen sulfatierten Polyol-, Polyamin- oder (Poly)amin-(poly)ol-Rest bedeutet, der gegebenenfalls eine oder mehrere acetalisierte oder aminalisierte Carbonylfunktionen enthält.

15. Verfahren nach Anspruch 14, dadurch gekennzeichnet, daß die Repetiereinheiten IV mit acetalisierter oder aminalisierter Carbonylfunktion der Formel V 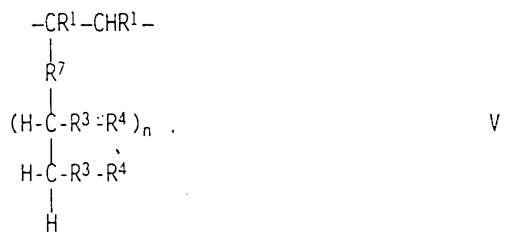 entsprechen, in der
R¹, R³, R⁴, R⁷ und n die für die Formel I angegebenen Bedeutungen haben; mit der Maßgabe, daß
(1) mindestens einmal, vorteilhaft ein- oder zweimal pro Molekül an demselben Kohlenstoffatom stehende Substituenten H und -R³-R⁴ zusammen mit diesem Kohlenstoffatom eine Carbonylfunktion C=O bilden, die durch eine Hydroxyl- bzw. Aminofunktion in 3-Stellung in bezug auf die Carbonylfunktion unter Bildung eines Tetrahydrofuran- bzw. Pyrrolidinringes oder durch eine Hydroxyl- bzw. Aminofunktion in 4-Stellung in bezug auf die Carbonylfunktion unter Bildung eines Pyran- bzw. Pentamethyleniminringes intramolekular acetalisiert bzw. aminalisiert ist, und daß (2) mindestens einer der Substituenten R⁴ ein Rest -SO₃⁻Na⁺ ist.

16. Verfahren nach einem der Ansprüche 11 bis 15, dadurch gekennzeichnet, daß die sauren Gruppen ganz oder teilweise neutralisiert sind.

17. Erzeugnisse, die aus einem nach dem Verfahren eines der Ansprüche 1 bis 16 hydrophil und bioaktiv beschichteten polymeren Substrat bestehen oder ein solches Substrat enthalten.

18. Verwendung der Erzeugnisse nach Anspruch 17 für technische, medizintechnische, hygienische oder biotechnische Zwecke.

19. Verwendung der Ezeugnisse nach Anspruch 17 als Katheter, Schläuche, Stents, Membranen, Wundverbände, Oxygenatoren, elastische Handschuhe für medizinische Zwecke oder Kondome.
